(19) Europäisches Patentamt
European Patent Office
Office européen des brevets

(11) Publication number: **0 102 195**
**A2**

(12) **EUROPEAN PATENT APPLICATION**

(21) Application number: 83304384.7

(22) Date of filing: 28.07.83

(51) Int. Cl.$^3$: C 07 D 451/04
C 07 D 451/14, A 61 K 31/46
//C07C143/78, C07C143/822,
C07D265/26

(30) Priority: 03.08.82 GB 8222345
11.06.83 GB 8316006

(43) Date of publication of application:
07.03.84 Bulletin 84/10

(84) Designated Contracting States:
CH DE FR GB IT LI NL

(71) Applicant: BEECHAM GROUP PLC
Beecham House Great West Road
Brentford Middlesex(GB)

(72) Inventor: King, Francis David
67 Cherry Garden Lane
Newport Essex(GB)

(72) Inventor: Martin, Roger Thomas
472 Vardon Road
Stevenage Hertfordshire(GB)

(72) Inventor: Wootton, Gordon
51 Parkway
Sawbridgeworth Hertfordshire(GB)

(74) Representative: Stott, Michael John et al,
European Patent Attorney Beecham Pharmaceuticals
Great Burgh Yew Tree Bottom Road
Epsom Surrey KT18 5XQ(GB)

(54) **Substituted aza bicycloalkyl derivatives with dopamine antagonist activity.**

(57) Compounds of formula (I):, or a pharmaceutically acceptable salt and/or N-oxide and/or a solvate thereof:

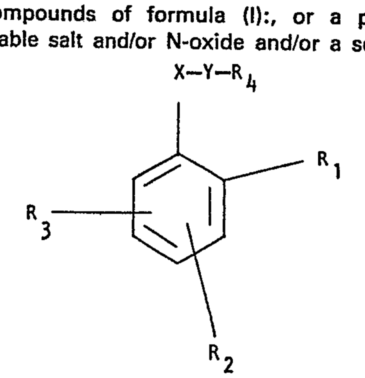

(I)

wherein:

one of X and Y is CO and the other is NH;

$R_4$ is a group:

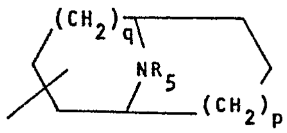

(II)

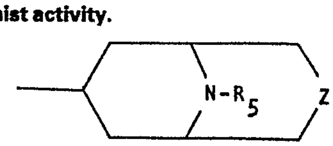

(III)

or

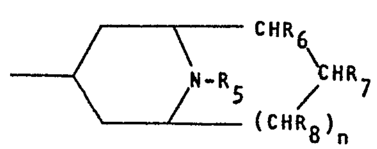

(IV)

wherein:

p and q each independently are 0 to 2;

Z is O or S;

n is 0 or 1;

one of $R_6$ and $R_7$ when n=0 is $C_{1-4}$ alkoxy, $C_{1-4}$ alkoxycarbonyl, hydroxy or $C_{1-4}$ alkyl optionally substituted by hydroxy, $C_{1-4}$ alkoxy or $C_{1-4}$ acyloxy, and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl;

./...

EP 0 102 195 A2

Croydon Printing Company Ltd.

$R_5$ is $C_{1-7}$ alkyl, $-(CH_2)_sR_{10}$, s being 0 to 2 and $R_{10}$ being $C_{3-8}$ cycloalkyl, $-(CH_2)_tR_{11}$, t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy;

$R_1$ is carboxylic $C_{1-7}$ acyl, carboxylic $C_{1-7}$ acylamino optionally N-substituted by $C_{1-6}$ alkyl, carboxylic $C_{1-7}$ acyloxyamino, $C_{1-6}$ alkylsulphonyl, $C_{1-6}$ alkylsulphinyl, halogen, $C_{1-6}$ alkyl, $C_{3-6}$ cycloalkyl or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups any of which phenyl moieties may be substituted by one or more halogen, trifluoromethyl, $C_{1-6}$ alkoxy or nitro groups, or amino-disubstituted by $C_{4-5}$ polymethylene; and

$R_2$ and $R_3$ are the same or different and are hydrogen, halogen, trifluoromethyl, $C_{1-6}$ alkoxy, $C_{1-6}$ alkylthio, carboxylic $C_{1-7}$ acyl, carboxylic $C_{1-7}$ acylamino, $C_{1-6}$ alkylsulphonyl, $C_{1-6}$ alkylsulphinyl, nitro or $NR_{12}R_{13}$, $NR_{12}R_{13}CO$, $NR_{12}R_{13}SO_2$, or $C_{1-6}$ alkyl-$SO_2NR_{14}$, $NR_{12}R_{13}SO_2NR_{14}$ wherein $R_{12}$ and $R_{13}$ are the same or different and are hydrogen, $C_{1-6}$ alkyl, $C_{3-8}$ cycloalkyl, phenyl or phenyl $C_{1-4}$ alkyl groups any of which phenyl moieties may be substituted by one or more halogen, trifluoromethyl, $C_{1-6}$ alkoxy or nitro groups, or $R_{12}$ and $R_{13}$ together form $C_{4-5}$ polymethylene, and $R_{14}$ is hydrogen or $C_{1-6}$ alkyl having anti-hypertensive activity, a process for their preparation and their use as pharmaceuticals.

0102195

B1311/1487

TITLE MODIFIED
see front page

NOVEL BENZAMIDES AND ANILIDES

This invention relates to novel compounds, to a process for their preparation and their use.

U.S. Patent No. 4273778 and published European Application No. 80304467.6 (corresponding to allowed U.S. Application No. 213237) disclose benzamides and anilides respectively having a bicyclic side chain. These compounds are described as having dopamine antagonist activity.

A novel class of benzamides and anilides have been discovered which compounds also have dopamine antagonist activity.

Accordingly, the present invention provides a compound of formula (I), or a pharmaceutically acceptable salt and/or N-oxide and/or a solvate thereof:

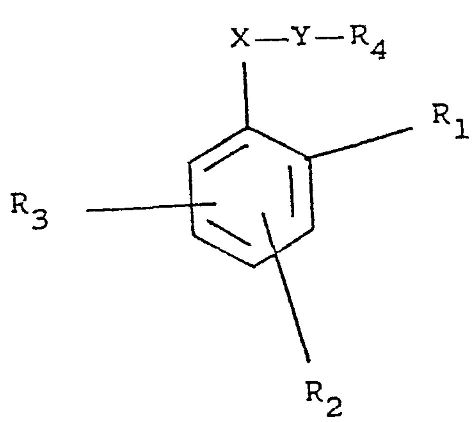

(I)

wherein:

one of X and Y is CO and the other is NH;

$R_4$ is a group:

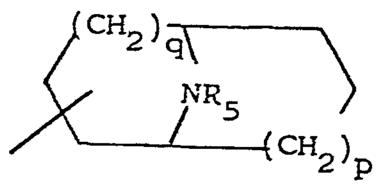

(II)

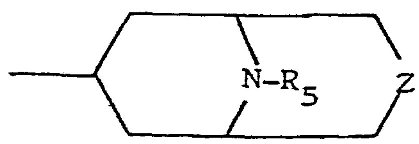

(III)

or

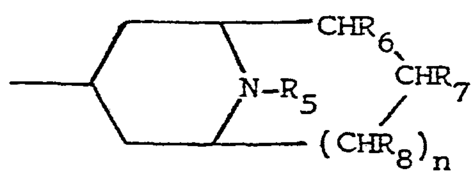

(IV)

wherein:

p and q each independently are 0 to 2;

Z is O or S;

n is 0 or 1;

one of $R_6$ and $R_7$ when n=0 is $C_{1-4}$ alkoxy, $C_{1-4}$ alkoxycarbonyl, hydroxy or $C_{1-4}$ alkyl optionally substituted by hydroxy, $C_{1-4}$ alkoxy or $C_{1-4}$ acyloxy, and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl;

$R_5$ is $C_{1-7}$ alkyl, - $(CH_2)_sR_{10}$,s being O to 2 and $R_{10}$ being $C_{3-8}$ cycloalkyl, $-(CH_2)_tR_{11}$,t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy;

$R_1$ is carboxylic $C_{1-7}$ acyl,carboxylic $C_{1-7}$ acylamino optionally N-substituted by $C_{1-6}$ alkyl, carboxylic $C_{1-7}$ acyloxyamino, $C_{1-6}$ alkylsulphonyl, $C_{1-6}$ alkylsulphinyl, halogen, $C_{1-6}$ alkyl, $C_{3-6}$ cycloalkyl or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups any of which phenyl moieties may be substituted by one or more halogen, trifluoromethyl, $C_{1-6}$ alkoxy or nitro groups, or amino-disubstituted by $C_{4-5}$ polymethylene; and

$R_2$ and $R_3$ are the same or different and are hydrogen, halogen, trifluoromethyl, $C_{1-6}$ alkoxy, $C_{1-6}$ alkylthio, carboxylic $C_{1-7}$ acyl, carboxylic $C_{1-7}$ acylamino, $C_{1-6}$ alkylsulphonyl, $C_{1-6}$ alkylsulphinyl, nitro or $NR_{12}R_{13}$, $NR_{12}R_{13}CO$, $NR_{12}$ $R_{13}SO_2$, or $C_{1-6}$ alkyl-$SO_2NR_{14}$, $NR_{12}R_{13}SO_2NR_{14}$ wherein $R_{12}$ and $R_{13}$ are the same or different and are hydrogen, $C_{1-6}$ alkyl, $C_{3-8}$ cycloalkyl, phenyl or phenyl $C_{1-4}$ alkyl groups any of which phenyl moieties may be substituted by one or more halogen, trifluoromethyl, $C_{1-6}$ alkoxy or nitro groups, or $R_{12}$ and $R_{13}$ together form $C_{4-5}$ polymethylene, and $R_{14}$ is hydrogen or $C_{1-6}$ alkyl.

Preferably, X is CO and Y is NH.

A preferred value of $R_4$ is of formula (II).

When $R_4$ is a group of formula (II) as defined, p is suitably 0 or 1, and q is suitably 0 or 1.

Often the group Y and the heterobicycle nitrogen atom are separated by 2 or 3 carbon atoms preferably 3.

The XY moiety is preferably in an equatorial orientation to the heterobicycle ring.

When $R_4$ is a group of formula (III) as defined Z is O or S, preferably O.

When $R_4$ is a group of formula (IV) as defined, preferably each of $R_6$ and $R_7$ when n=0 and each of $R_6$, $R_7$ and $R_8$ when n=1 are in the exo-position.

Suitable examples for one of $R_6$ and $R_7$ when n=0 include methoxy, ethoxy, n-propoxy, methyl, ethyl, n-propyl, methoxycarbonyl and ethoxycarbonyl, hydroxy, hydroxymethyl or hydroxethyl, methoxymethyl, methoxyethyl, ethoxymethyl, ethoxyethyl, acetylmethyl and acetylethyl. Methyl is preferred.

Suitable examples for one of $R_6$, $R_7$ and $R_8$ when n=1 include methyl, ethyl and n-propyl.

Suitable examples for the other of $R_6$ and $R_7$ when n=0 and for the other two of $R_6$, $R_7$ and $R_8$ when n=1 include hydrogen, methyl, ethyl and n- and iso-propyl.

Examples of $R_5$ when $C_{1-7}$ alkyl include methyl, ethyl and n-and iso-propyl. Within $C_{1-7}$ alkyl, $C_{4-7}$ alkyl are of interest, especially those of the formula $(CH_2)_uR_{16}$ wherein u is 1 or 2 and $R_{16}$ is a secondary or

tertiary $C_{3-6}$ alkyl group. Examples of $C_{4-7}$ alkyl include n-,sec- and tert-butyl, n-pentyl, n-heptyl, and especially iso-butyl, 3-methylbutyl, 2,2-dimethylpropyl and 3,-3-dimethylbutyl.

Preferred examples of $R_5$, when $-(CH_2)_s R_{10}$ are those wherein s is 1 or 2, in particular those wherein $R_{10}$ is $C_{5-8}$ cycloalkyl, such as cyclohexyl, and cyclopropyl.

Preferred examples of $R_5$ , when $-(CH_2)_tR_{11}$, are those wherein t is 1. $R_{11}$ may be 2- or 3-thienyl or preferably is phenyl optionally substituted by one of $C_{1-4}$ alkoxy, trifluoromethyl, halogen, carboxy, esterified carboxy or $C_{1-4}$ alkyl optionally substituted by hydroxy, $C_{1-4}$ alkoxy, carboxy, esterified carboxy and in vivo hydrolysable acyloxy.

When phenyl is substituted by optionally substituted $C_{1-4}$ alkyl, examples of $C_{1-4}$ alkyl include methyl, ethyl, n- and iso-propyl, and n-, iso-, sec- and tert- butyl; methyl however is preferred. Examples of substituents of such alkyl groups include hydroxy, methoxy, ethoxy, n- and iso- propoxy, carboxy, esterified carboxy and in vivo hydrolysable acyloxy. The substitution preferably occurs on the terminal carbon atom of the alkyl group.

Examples of esterified carboxy groups include $C_{1-4}$ alkoxycarbonyl, such as methoxy-, ethoxy-, n- and isopropoxycarbonyl, phenoxycarbonyl or benzyloxycarbonyl, either being optionally substituted in the phenyl ring by one or two substituents selected from $C_{1-4}$ alkyl, $C_{1-4}$ alkoxy, trifluoromethyl, halogen or nitro.

Examples of _in vivo_ hydrolysable acyloxy groups include $C_{1-6}$ alkanoyloxy, for example acetoxy, propionoxy, _n_- and _iso_- butyroxy, and 2,3-dimethylpropanyloxy, benzyloxy or benzenesulphonyloxy either being optionally substituted in the phenyl ring by one or two substituents selected from $C_{1-4}$ alkyl, $C_{1-4}$ trifluoromethyl, halogen or nitro, or other sulphonyloxy groups, for example $C_{1-6}$ alkanesulphonyloxy group, such as methanesulphonyloxy.

The most preferred examples of $R_5$, when $-(CH_2)_tR_{11}$, are those wherein t is 1 and $R_{11}$ is unsubstituted phenyl or monosubstituted phenyl in particular mono-p-substituted phenyl. Examples of preferred p-substitutents include methyl, trifluoromethyl, fluoro, chloro and bromo, especially fluoro. Unsubstituted benzyl, p-fluorobenzyl, p-chlorobenzyl and p-methylbenzyl are especially preferred examples of $R_5$.

Suitable examples of $R_1$ include formyl, acetyl, propionyl, n- and iso-butyryl, formylamino, acetylamino, propionylamino, n- and iso-butyrylamino, acetoxyamino, propionylamino, n- and iso-butryloxyamino, methylsulphonyl, methylsulphinyl, chloro, bromo, methyl, ethyl, n- and iso-propyl, n,-sec- and tert-butyl, cyclopropyl, cyclobutyl, cyclopentyl, cyclohexyl, or amino optionally substituted by one or two methyl, ethyl, n- or iso-propyl, n-, sec- or tert-butyl groups, cyclopropyl, cyclopentyl, cyclohexyl, phenyl or benzyl groups or N-disubstituted by $C_4$ or $C_5$ polymethylene.

Favoured values for $R_1$ include acetylamino and amino optionally substituted as defined. Preferably substituted amino, most preferably amino monosubstituted by methyl.

Suitable examples of $R_2$ and $R_3$ include the following groups: hydrogen, chloro, bromo,$CF_3$, methoxy, ethoxy, n- and iso-propoxy, methylthio, ethylthio, n- and iso- propylthio, nitro, amino, $C_{1-4}$ alkanoylamino such as formylamino, acetylamino, propionylamino, n- and iso-butyrylamino, aminosulphonyl; and amino, aminosulphonyl and aminosulphonylamino N-substituted by one or two methyl, ethyl, n- or iso-propyl n-, sec-, or tert-butyl; cyclopropyl, cyclopentyl, cyclohexyl, phenyl or benzyl groups or N-disubstituted by $C_4$ or $C_5$ polymethylene; or aminosulphonylamino or methyl-, ethyl-, n- or iso-propylsulphonylamino.

Preferably $R_2$ and $R_3$ are independently hydrogen, chloro, amino or optionally substituted aminosulphonyl, as defined.

When $R_2$ and $R_3$ are other than $C_{1-6}$ alkoxy or $C_{1-6}$ alkylthio, $R_2$ is preferably in the 4-position with respect to X as the 1-position; $R_3$ is then preferably in the 5-position similarly defined.

It will of course be realised that some of the compounds of the formula (I) have chiral or prochiral centres, and thus are capable of existing in a number of stereoisomeric forms. The invention extends to each of these stereoisomeric forms, and to mixtures thereof (including racemates). The different stereoisomeric forms may be separated one from the other by the usual methods, or any given isomer may be obtained by sterospecific or asymmetric synthesis.

Preferred compounds of formula (I) include those containing the moiety of formula (V):

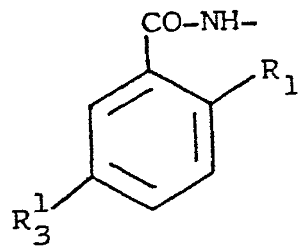

(V)

wherein $R_3{}^1$ is aminosulphonyl optionally substituted as defined and $R_1$ is as defined in formula (I).

A preferred group of compounds within those of formula (I) is therefore of formula (VI):

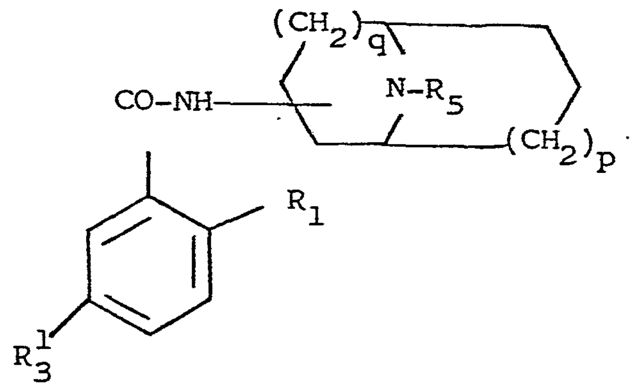

(VI)

wherein the variable groups are as hereinbefore defined. Suitable values for $R_3{}^1$ N-substituents are as described under formula (I). Preferably $R_3{}^1$ is aminosulphonyl optionally substituted by one or two methyl groups. Suitable and preferred values for $R_1$ are as so described under formula (I).

More suitably p is 0 or 1, it is believed preferably 0. Preferably q is 1 and the CONH moiety is then attached at the 3-position (conventional numbering) and in the β orientation.

A sub-group of compounds within those formula (VI) those of formula (VII):

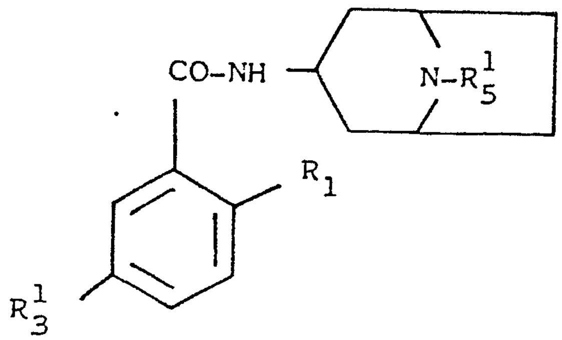

(VII)

wherein:
$R_5^1$ is $C_{4-7}$ alkyl or $-(CH_2)_5R_{10}$ where s is 0 to 2 and $R_{10}$ is $C_{5-8}$ cycloalkyl; and the remaining variables are as defined in formula (V).

Suitable and preferred $R_1$ are as so described under formula (I). Preferably $R_1$ is amino or substituted amino as defined.

Suitable and preferred $R_3^1$ are as so described under formula (I) for corresponding $R_3$.

A group of compounds within those of formula (VII) is that wherein $R_5^1$ is $(CH_2)_uR_{16}$ as defined or $(CH_2)_uR_{10}$ where u and $R_{10}$ are as defined.

Examples of $R_5^1$ in these compounds include iso-butyl, 3-methylbutyl, 3,3-dimethylbutyl, 2,2-dimethylpropyl and cyclopropylethyl.

It is preferred that the CONH moiety is in the β-orientation to the nortropane ring.

A sub-group of compounds within those of formula (VI) is of the formula (VIII):

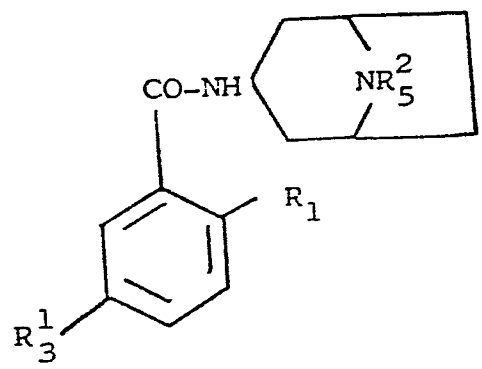

(VIII)

wherein:

$R_5^2$ is thienylmethyl or $-(CH_2)_tR_{11}$, t being 1 or 2, and $R_{11}$ being phenyl optionally subsituted by one or two substituents selected from $C_{1-4}$ alkoxy, trifluoromethyl, halogen, carboxy, esterified carboxy, and $C_{1-4}$ alkyl optionally substituted by hydroxy, $C_{1-4}$ alkoxy, carboxy esterified carboxy or in vivo hydrolysable acyloxy.

Particularly preferred compounds are those wherein $R_5^2$ is benzyl optionally substituted in the phenyl ring by one or two substituents selected from $C_{1-4}$ alkoxy, trifluoromethyl, halogen and $C_{1-4}$ alkyl.

It is especially preferred that the phenyl ring is unsubstituted.

Suitable and preferred $R_1$ are as so described under formula (I). Preferably $R_1$ is substituted amino.

Suitable and preferred $R_3^1$ are as so described under formula (V).

It is preferred that the CONH moiety is in the β-orientation to the nortropane moiety.

A third sub-group of compounds within those of formula (VI) is of formula (IX):

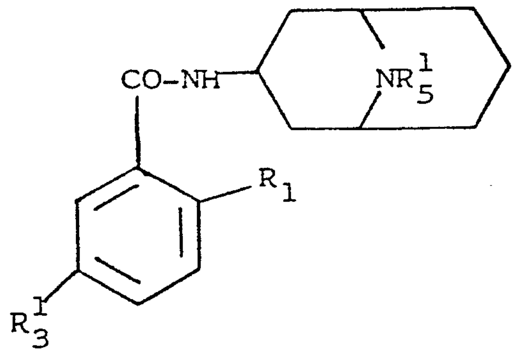

(IX)

wherein $R_5^1$ is as defined in formula (VII).

Suitable and preferred variables are as so described under formula (VII).

Another sub-group of compounds within those of the formula (VI) of interest are those of the formula (X):

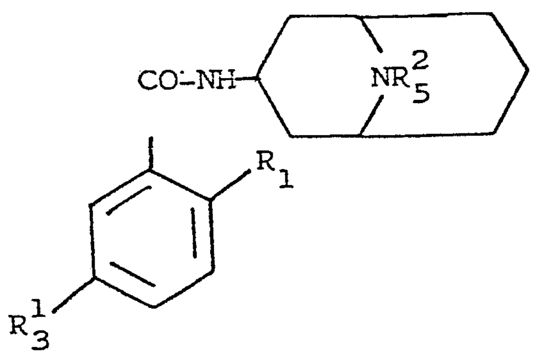

(X)

wherein $R_5^2$ is as defined in formula (VIII).

Preferred compounds are those wherein $R_5^2$ is optionally substituted benzyl as defined under formula (VIII).

It is especially preferred that the phenyl ring is mono-substituted and/or that the substitution is in the para-position and/or that the substituent is chloro, fluoro or methyl.

Suitable and preferred $R_3^1$ are as so described under formula (VIII).

A second group of compounds within those of formula (I) is of formula (XI):

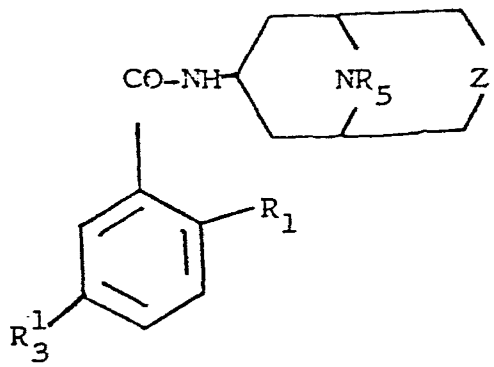

(XI)

wherein the variables are as defined in formulae (I) and (III).

Suitable and preferred variables are as so described under formula (VI).

Preferably Z is O, and the CONH moiety is in the β-orientation.

There are sub-groups within formulae (VI) to (XI) respectively wherein $R_1$ is $C_{1-7}$ acylamino or substituted amino as defined.

There are further sub-groups within formulae (VI) to (XI) respectively wherein $R_1$ is halogen or $C_{1-6}$ alkyl.

There are further compounds within formula (I) containing the moiety of formula (XII):

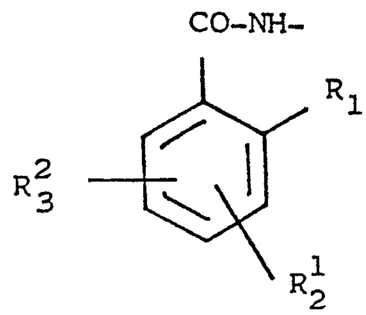

(XII)

wherein $R_2{}^1$ and $R_3{}^2$ are the same or different and are hydrogen, halogen, hydroxy, $C_{1-6}$ alkoxy, $C_{1-6}$ alkylthio, trifluoromethyl, amino or $C_{1-7}$ acylamino; and

$R_1$ is as hereinbefore defined.

Another group of compounds within formula (I) is therefore of formula (XIII):

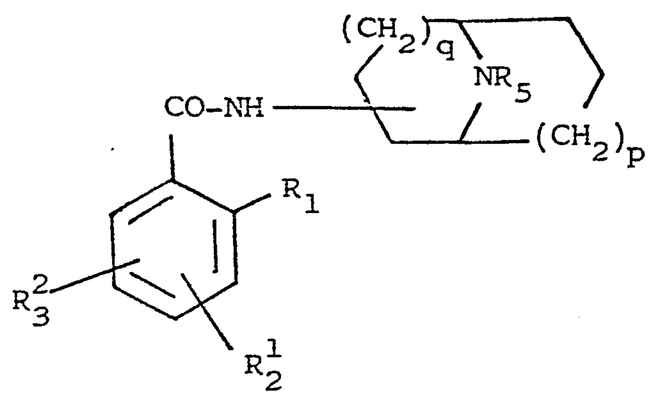

(XIII)

wherein the variable groups are as hereinbefore defined.

Suitable and preferred values for $R_2{}^1$ and $R_3{}^2$ are as so described for the relevant $R_2$ and $R_3$ under formula (I).

Suitable values for $R_1$ are as so described under formula (I).

A sub-group of compounds within those of formula (XIII) are those of formula (XIV):

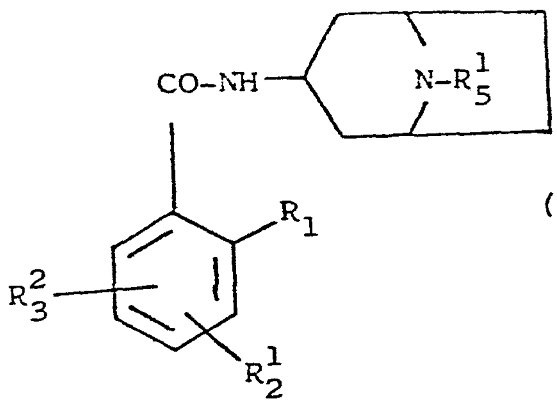

(XIV)

wherein the variable groups are as hereinbefore defined.

Suitable and preferred $R_1$ are as so described under formula (I).

Suitable and preferred $R_2{}^1$ and $R_3{}^2$ are as so described under formula (I) for corresponding $R_2$ and $R_3$.

A group of compounds within those of formula (XIV) is that wherein $R_3{}^1$ is $(CH_2)_uR_{10}$ where u and $R_{10}$ are as defined.

Examples of $R_5{}^2$ in these compounds include iso-butyl, 3-methylbutyl, 2,2-dimethylpropyl, 3,3-dimethylbutyl and cyclopropylethyl.

It is preferred that the CONH moiety is in the β-orientation to the nortropane ring.

A sub-group of compounds within those of formula (XIII) is of formula (XV):

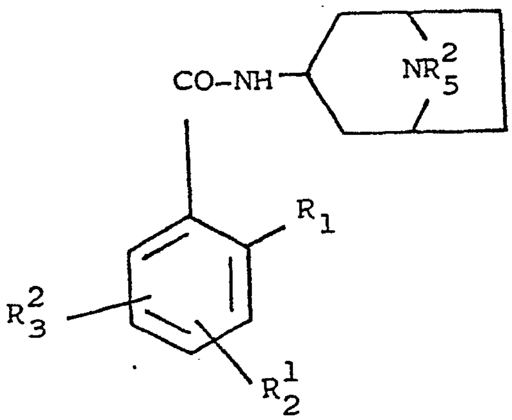

(XV)

wherein the variable groups are as hereinbefore defined.

Particularly preferred compounds are those wherein $R_5^2$ is benzyl optionally substituted in the phenyl ring by one or two substituents selected from $C_{1-4}$ alkoxy, trifluoromethyl, halogen and $C_{1-4}$ alkyl.

Suitable and preferred $R_1$ are as so described under formula (I).

Suitable and preferred $R_2^1$ and $R_3^2$ are as so described under formula (V).

It is preferred that the CONH moiety is in the β-orientation to the nortropane moiety.

A third sub-group of compounds within those of formula (XIII) is of formula (XVI):

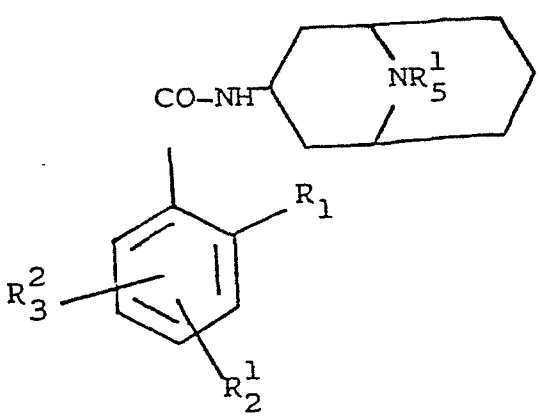

(XVI)

wherein the variable groups are as hereinbefore defined under formula (XIV).

Another sub-group of compounds within those of the formula (XIII) of interest are those of formula (XVII):

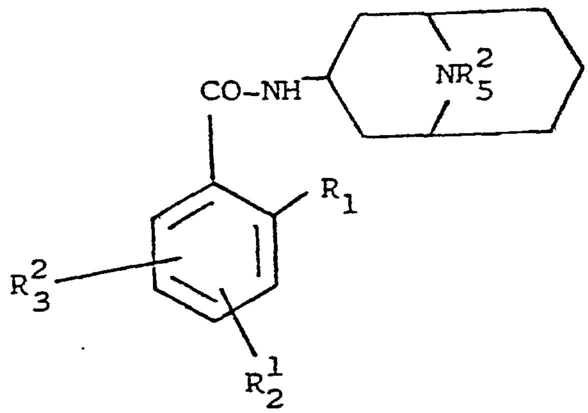

(XVII)

wherein $R_5^2$ is as defined in formula (VIII).

Preferred compounds are those wherein $R_5^2$ is optionally substituted benzyl as defined under formula (VIII).

It is especially preferred that the phenyl ring is mono-substituted and/or that the substitution is in the para-position and/or that the substituent is chloro, fluoro or methyl.

Suitable and preferred $R_2^1$ and $R_3^2$ are as so described under formula (XIV).

These are sub-groups within formulae (XIV) to (XVII) respectively wherein $R_1$ is $C_{1-7}$ acylamino or substituted amino as defined.

There are further sub-groups within formulae (XIV) to (XVII) respectively wherein $R_1$ is halogen or $C_{1-6}$ alkyl.

Within each of these latter sub-groups there are sub-groups wherein the benzamide moiety is of formula (XVIII):

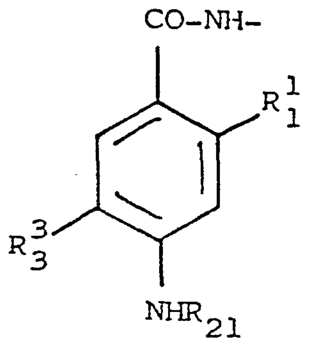

(XVIII)

wherein:

$R_1^1$ is halogen or $C_{1-6}$ alkyl;
$R_3^3$ is halogen; and
$R_{21}$ is hydrogen or $C_{1-4}$ alkanoyl.

The invention also provides a process for the preparation of a compound of the formula (I), which process comprises reacting a compound of the formula (XIX):

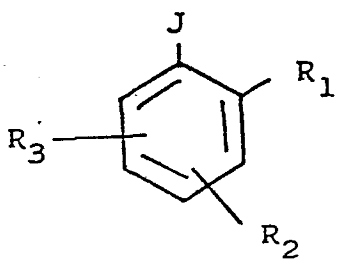

(XIX)

with a compound of formula (XX):

$L-R_4$

wherein:

One of J and L is COQ, where Q is a leaving group, and the other is $-NH_2$; and the remaining variable groups are as defined in formula (I), with the proviso that when J is $-NH_2$, $R_1$, $R_2$ or $R_3$ is other than amino,

and thereafter optionally converting $R_1$, $R_2$ or $R_3$ to another $R_1$, $R_2$ or $R_3$ respectively; as necessary converting $R_5$ to other $R_5$; and optionally forming a pharmaceutically acceptable salt of the resultant compound of the formula (I).

The leaving group Q is a group that is readily displaceable by a nucleophile. Examples of such groups are hydroxy, halogen such as chloro and bromo, acyloxy such as $C_{1-4}$ alkanoyloxy, $C_{1-4}$alkoxycarbonyloxy and activiated hydrocarbyloxy such as pentachlorophenoxy. Another example of an acyloxy group Q is when Q is joined to $R_1$ to form -O-CO-NG- wherein G is hydrogen or an $R_1$ amino substituent in the resultant compound of formula (I).

If the leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as -10 to 100°C, for example 0 to 80°C.

If the leaving group is a halide, then the reaction is preferably carried out at a non-extreme temperature in an inert non-hydroxylic solvent, such as dichloromethane, benzene, toluene or diethyl ether. It is also preferably carried out in the presence of an acid acceptor, such as an organic base, in particular a tertiary amine such as triethylamine, trimethylamine, pyridine or picoline, some of which can also function as the solvent. Alternatively, the acid acceptor can be inorganic, such as calcium carbonate, sodium carbonate or potassium carbonate.

If the leaving group is acyloxy, then the reaction is preferably carried in substantially the same manner as if the leaving group were hydroxy. Suitable examples of acyloxy leaving groups include $C_{1-4}$ alkanoyloxy, mesyloxy, tosyloxy and triflate; and when $R_1$ is optionally monosubsituted amino, and X-Y is CO-NH, -O-CO-NG- as defined.

If the leaving group is $C_{1-4}$ alkoxycarbonyloxy, then the reaction is preferably carried out in an inert solvent, such as methylene chloride, at a non-extreme temperature in the presence of an acid acceptor, such as triethylamine.

If the leaving group is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

Preferably Q is halogen, such as chloro.

The compounds of formula (XIX) and (XX) are either known compounds or can be prepared analogously to the preparation of structurally similar known compounds.

Compounds of formula (XIX) wherein $R_3$ is aminosulphonyl may be formed from the corresponding $R_3$ chlorosulphonyl derivatives of the compound of formula (XIX) wherein $R_3$ is hydrogen, with a suitable amine or ammonia.

The skilled man will appreciate that the choice or necessity of conversion of groups $R_1$, $R_2$ and/or $R_3$ to other groups $R_1$, $R_2$ and/or $R_3$ will be dicatated by the nature and position of substituents $R_1$, $R_2$ and $R_3$.

It will be apparent that compounds of the formula (I) containing an $R_1$, $R_2$, $R_3$ or $R_5$ group which is convertible to another $R_1$, $R_2$ and $R_3$ group or to an $R_5$ group are useful novel intermediates. A number of such conversions is possible not only for the end compounds of fomula (I), but also for their intermediates as follows:

(a) an hydrogen substituent is convertible to a nitro substituent by nitration;

(b) a nitro substituent is convertible to an amino substituent by reduction;

(c) a $C_{1-4}$ acylamino substituent is convertible to an amino substituent by deacylation;

(d) an amino substituent is convertible to a $C_{1-4}$ acylamino substituent by acylation;

(e) a hydrogen substituent is convertible to a halogen substituent by halogenation;

(f) a $C_{1-6}$ alkylthio or $C_{1-6}$ alkylsulphinyl substituent is convertible to a $C_{1-6}$ alkylsulphinyl or a $C_{1-6}$ alkylsulphonyl substituent respectively by oxidation; and

(g) an amino substituent is convertible to a $C_{1-6}$ alkyl $SO_2NR_{14}$ or $NR_{12}R_{13}$ $SO_2NR_{14}$ substituent by reaction with $C_{1-6}$ alkyl $SO_2NQ_2$ or $NR_{12}R_{13}SO_2Q_2$ where $Q_2$ is leaving group.

(h) a fluoro or chloro substituent is convertible to an optionally substituted amino substituent by reaction with a suitable amine or ammonia.

Conversions (a) to (h) are only exemplary and are not exhaustive of the possibilities.

In regard to (a), nitration is carried out in accordance with known procedures.

In regard to (b), the reduction is carried out in accordance with known procedures, for example, with a reagent suitable for reducing nitroanisole to aminoanisole.

In regard to (c), deacylation is carried out by treatment with a base, such as an alkali metal hydroxide.

In regard to (d), the acylation is carried out with an acylating agent, such as the corresponding acid or acid chloride. Formylation is carried out with the free acid.

In regard to (e), halogenation is carried out with conventional halogenating agents.

In regard to (f), oxidation is carried out at below ambient temperatures in a non-aqueous solvent, such as a chlorinated hydrocarbon, in the presence of an organic peracid, such as 3-chloroperbenzoic acid, or in water in the presence of a soluble strong inorganic oxidant, such as an alkali metal permanganate or in aqueous hydrogen peroxide.

In regard to (g), $Q_2$ is often halide, for example chloride or bromide, or hydroxy. When halide is the leaving group, the reaction is generally carried out in the presence of a base. When hydroxy is the leaving group, the reaction is generally carried out in the presence of a dehydrating agent, such as dicyclohexylcarbodiimide, in an inert sovent at non-extreme temperature, such as ambient temperature.

In regard to (h), the amination is carried out under conventional conditions using an inert solvent such as $CH_2Cl_2$ or an excess of amine also functioning as the solvent.

Depending on steric and/or electronic factors and/or reaction conditions mono- or di-acylation may occur. If diacylation takes place, subsequent selective monodeacylation of the resultant compound is necessary to form the desired alkyl- or aminosulphonylamino group.

Treatment with a base such as methanolic sodium hydroxide at ambient or slightly elevated temperatures is apt.

It is often most convenient to affect the coupling of compounds (XIX) and (XX) using a compound of formula (XIX) wherein $R_1$ is chloro or fluoro, preferably fluoro and to convert the relevant minor group in the coupling product to optionally substituted amino.

Similarly, it is often most convenient to effect the coupling of compounds (XIX) and (XX) using a compound of formula (XIX) wherein one of $R_2$ and $R_3$ is amino and to convert alkyl- or aminosulphonylamino.

It will be appreciated that, $R_5$ when optionally substituted benzyl as hereinbefore defined, may be replaced by another group $R_5$.

Such $R_5$ benzyl groups may be removed for example when $R_1$, $R_2$ or $R_3$ is not halogen by conventional transition metal catalysed hydrogenolysis to give compounds of the formula (XXI):

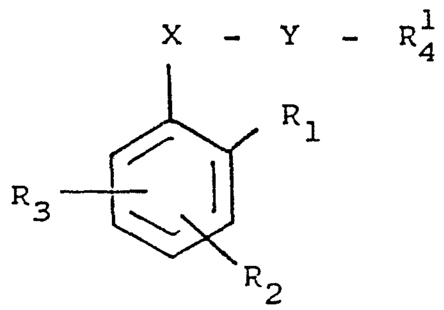

(XXI)

wherein $R_4^1$ is $R_4$ wherein the $R_5$ substituent is replaced by hydrogen and the remaining variable groups are as defined in formula (I).

This invention also provides an optional process step in the preparation of a compound of the formula (I) which comprises the reaction of a corresponding compound of the formula (XXI) as hereinbefore defined with a compound $Q_3R_5$ wherein $R_5$ is as defined in formula (I) and $Q_3$ is a leaving group, and optionally forming a pharmaceutically acceptable salt of the resulting compound of the formula (I).

Suitable values for $Q_3$ include groups readily displaced by nucleophiles such as Cl, Br, I, $OSO_2CH_3$ or $OSO_2C_6H_4\underline{p}CH_3$.

Favoured values for $Q_3$ include Cl, Br and I.

Particularly suitably the compound $Q_3R_5$ is a benzyl halide, such as the bromide or chloride.

The reaction may be carried out under conventional alkylation conditions for example in an inert solvent such as dimethylformamide in the presence of an acid acceptor such as potassium carbonate. Generally the reaction is carried out at non-extreme temperature such as at ambient or at a slightly elevated temperature.

Converting $R_5$ to another $R_5$ in the compound of the formula (XX) before coupling with the compound of the formula (XIX) or its derivative is preferred. Such interconversions are effected conveniently under the above conditions. It is desirable to protect the amine function with a group readily removable by hydrolysis such as a $C_{2-7}$ alkanoyl group before $R_5$ interconversion.

The substituents in the phenyl ring when $R_5$ is benzyl in a compound of formula (I), in particular the substituted $C_{1-4}$ alkyl substituents, are interconvertible. A number of such interconversions are possible not only for the end compounds of formula (I), but also for their intermediates as follows:

(i) a carboxy $C_{1-4}$ alkyl substituent is convertible to an esterified carboxy $C_{1-4}$ alkyl substituent by esterification:

(ii) an esterified carboxy $C_{1-4}$ alkyl substituent is convertible to a carboxy $C_{1-4}$ alkyl substituent by deesterification;

(iii) $C_{1-4}$ alkoxy $C_{1-4}$ alkyl substituent or an in vivo hydrolysable $C_{2-4}$ acyloxy $C_{1-4}$ alkyl substituent is convertible to an hydroxy $C_{1-4}$ alkyl substituent;

(iv) an optionally esterified carboxy or carboxy $C_{1-3}$ alkyl substituent is convertible to an hydroxymethyl or hydroxy $C_{2-4}$ alkyl substituent by reduction; and

(v) a hydroxy $C_{1-4}$ alkyl is convertible to $C_{1-4}$ alkyl by O-alkylation or in vivo hydrolysable $C_{2-4}$ acyloxy $C_{1-4}$ alkyl by O-acylation.

Conversions (i) to (iv) are only exemplary and are not exhaustive of the possibilities.

In regard to (i) and (ii), the esterification and de-esterification reactions are carried out in conventional manner.

In regard to (iii), a $C_{1-4}$ alkoxy $C_{1-4}$ alkyl substituent is convertible to an hydroxy $C_{1-4}$ alkyl substituent by conventional methods, such as warming with aqueous hydrobromic acid or by treatment with pyridine hydrochloride, boron tribromide, boron triodide or iodotrimethylsilane.

As in vivo hydrolysable $C_{2-4}$ acyloxy $C_{1-4}$ alkyl substituent is convertible to an hydroxy $C_{1-4}$ alkyl substituent by acid or base hydrolysis.

In regard to (iv), the reduction is carried out with a selective metal complex hydride, for example lithium aluminium hydride, under conventional conditions.

In regard to (v), O-alkylation is carried out under conventional conditions in an inert solvent at a non-extreme temperature such as ambient temperature or slightly above or at reflux temperature. The $C_{1-4}$ alkylating agent has a leaving group that is readily displaceable by a nucleophile. Examples of leaving groups include halide, such as chloride, bromide or iodide, or labile acyloxy groups, such as mesyl or tosyl.

O-acylation is carried out under conventional conditions with an acylating agent which has an acyl group capable of forming an in vivo hydrolysable acyloxy group and a leaving group, such as halide, for

example chloride and bromide, and hydrogen. When halide is the leaving group, the reaction is generally carried out in the presence of a base. When hydroxy is the leaving group, the reaction is generally carried out in the presence of a dehydrating agent, such as dicylohexylcarbodiimide, in an inert solvent at non-extreme temperature, such as ambient temperature or slightly above, or reflux temperature.

Before carrying out any of these conversions, the effect, if any, on other substituents should be considered, and such reagents as are appropriate should be selected together with the adoption of such precautionary measures as are necessary. For example, O-alkylation and O-acylation may also produce N-alkylated and N-acylated products respectively unless the nitrogen atom(s) is (are) previously protected. This may be conveniently achieved by carrying out the alkylation or acylation reaction in a strong acid, such as trifluoroacetic acid, which protonates, and thereby protects, the nitrogen atom(s).

Compounds of the formula (XXI) are novel intermediates and thus form as aspect of the present invention.

It will be realised that in the compound of the formula (I) the -X-Y-linkage may have an α or β orientation with respect to the ring of the bicyclic moiety to which it is attached. A mixture of α and β isomers of the compound of the formula (I) may be systhesised non-stereospecifically and the desired isomer separated conventionally therefrom eg by chromatography; or alternatively the α and β isomer may if desired by synthesised from the corresponding α or β form of the compound of the formula (XX).

Synthesis from the corresponding α or β isomer of the compound of the formula (XX) is in general preferred.

It will be appreciated that, when X is NH, Y is CO and n=O in the compounds of the formulae (I) or (XX), epimerisation of the CO-ring linkage to the energetically more favourable orientation often takes place readily in the presence of acid or base. In such cases if the less favoured isomer is desired, it is preferred to stereospecifically synthesise the isomer of the compound of the formula (XX) and to convert it to the required comound of the formula (I) under such conditions to avoid epimersation.

The α or β form of the compound of the formula (XX) may if desired be prepared by known stereospecific processes, such as those leading to the α or β isomers of the compound of the formula (XX) depicted in the Scheme and described in the Descriptions hereinafter.

Compounds of the formula (XX) are known from or are preparable by the methods disclosed in published European Patent Applications and U.S. Patents.

SCHEME 1

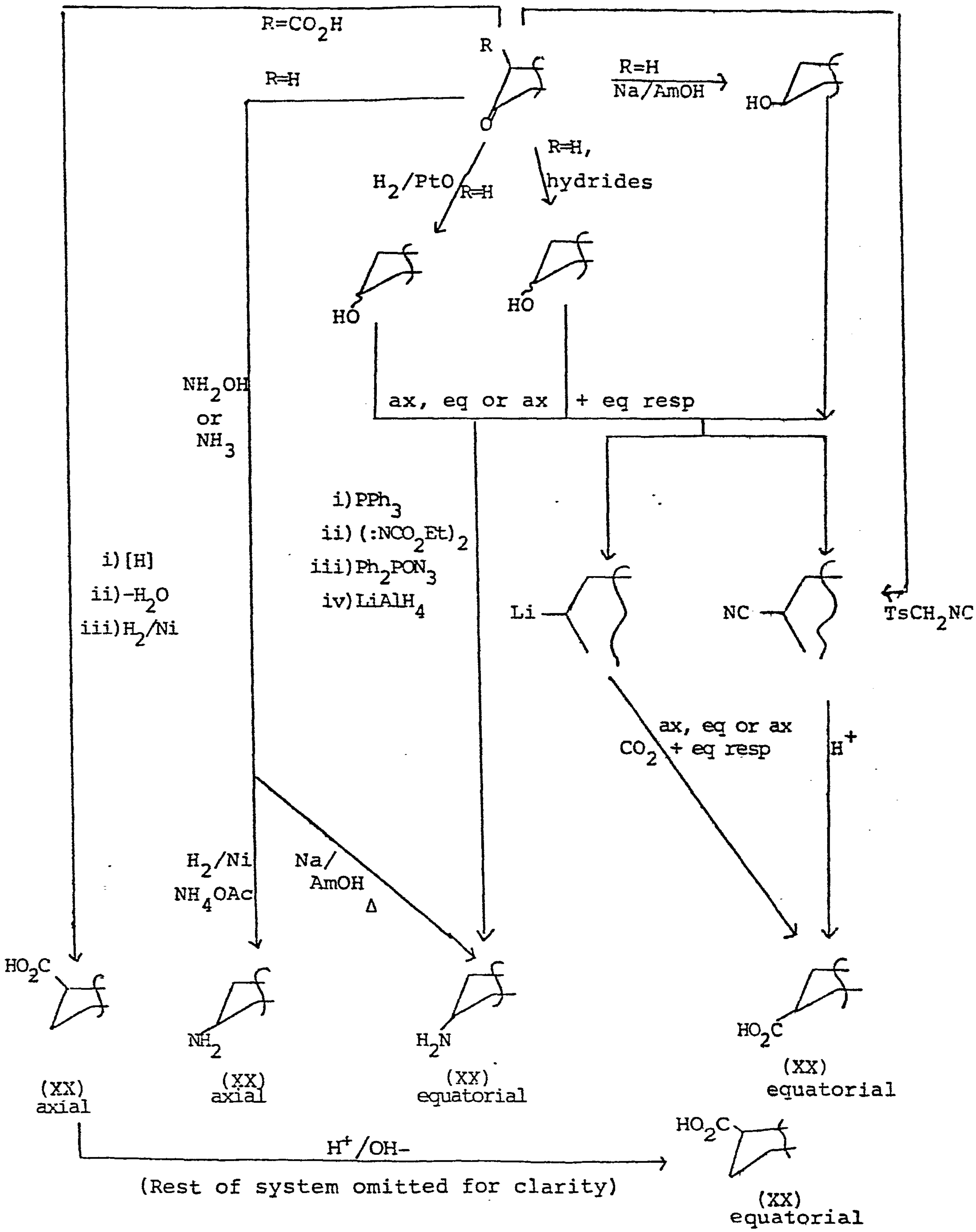
R=CO2H
R=H
R=H
Na/AmOH
R=H,
hydrides
H2/PtO
R=H
NH2OH
or
NH3
ax, eq or ax + eq resp
i) PPh3
ii) (:NCO2Et)2
iii) Ph2PON3
iv) LiAlH4
i) [H]
ii) -H2O
iii) H2/Ni
TsCH2NC
ax, eq or ax
CO2 + eq resp
H+
H2/Ni
NH4OAc
Na/
AmOH
Δ
(XX)
axial
(XX)
axial
(XX)
equatorial
(XX)
equatorial
H+/OH-
(Rest of system omitted for clarity)
(XX)
equatorial

Pharmaceutically acceptable salts, hydrates and N-oxides of the compounds of this invention may be formed conventionally. The salts may be formed for example by reaction of the base compound of formula (I) with a pharmaceutically acceptable organic or inorganic acid.

N-oxides of the nitrogen atom of the bicyclic ring system are produced by reaction of a compound of formula (I) with an organic peracid, such as m-chloroperbenzoic acid in, for example, a chlorinated hydrocarbon solvent at below ambient temperature.

Quaternary ammonium salts may be prepared by reaction of a compound of the present invention with the appropriate alkyl, aryl, aralkyl chloride, bromide or iodide. This reaction may be carried out in a solvent, such as acetone, methanol, ethanol, dimethylformamide at ambient or elevated temperature with or without pressure.

The compounds of the present invention are dopamine antagonists and may generally be used in the treatment of emesis. Depending on their balance between peripheral and central action on the nervous system, they may also be used in the treatment of disorders relating to impaired gastro-intestinal motility, such as retarded gastric emptying, dyspepsia, flatulence, oesophagal reflux and peptic ulcer and/or in the treatment of disorders of the central nervous system, such as psychosis.

1 Those compounds of the present invention which are of interest for their beneficial effect on gastric motility are the compounds of formula (VI) and the quaternary ammonium salts of the compounds of formula (I).

2 The invention also provides a pharmaceutical composition comprising a compound of formula (I), or a pharmaceutically acceptable salt thereof, or an solvate or N-oxide thereof, and a pharmaceutically acceptable carrier.

13 Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administerable compositions are preferred.

Tablets and capsules for oral administration are usually presented in a unit dose, and contain conventional excipients such as binding agents, fillers, tabletting agents, lubricants, disintegrants, and wetting agents. The tablets may be coated according to well known methods in the art. Oral liquid preparations are usually in the form of aqueous or oily suspensions, solutions, emulsions, syrups, or elixirs or are presented as a dry product for reconstitution with water or other vehicle before use. Such liquid preparations may contain conventional additives such as suspending agents, emulsifying agents, non-aqueous vehicles (which may include edible oils), preservatives, and flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in the vehicle and filter sterilizing before filling into a suitable vial or ampoule and sealing. Advantageously, adjuvants such as a local anaesthetic, preservatives and buffering agents are also dissolved in the vechicle.

Parenteral suspensions are prepared in substantially the same manner except that the compound is suspended in the vehicle instead of being dissolved and sterilized by exposure to ethylene oxide before suspending in the sterile vehicle. Advantageously, a surfactant or wetting agent is included in the composition to facilitate uniform distribution of the compound of the invention.

01 The invention further provides a method of treatment of disorders in mammals, such as humans, which comprises the administration of an effective amount of a compound of the formula (I), or a pharmaceutically acceptable salt thereof, or an hydrate or N-oxide thereof, or a pharmaceutical composition, as hereinbefore defined to the sufferer.

02 An amount effective to treat the disorders hereinbefore described depends on the relative efficacies of the compounds of the invention, the nature and severity of the disorder being treated and the weight of the mammal. However, a unit dose will normally contain 0.1 to 20 mg for example 0.5 to 10mg, of the compound of the invention. Unit doses will normally be administered more than once a day, for example 2,3,4,5 or 6 times a day such that the total daily dose is normally in the range 0.01 to 10mg/ kg per day. The compounds of the present invention have the ability to potentiate the effect of conventional analgesics in migraine treatment when administered concurrently with the analgesic.

03 Accordingly, the present invention also provides a pharmaceutical compositions comprising a compound of the formula (I) and an analgesic.

04 The compound of the formula (I) and the analgesic, such as aspirin or paracetamol, are present in the composition in amounts generally similar to their usual effective dose.

01 The composition can be a combination product, for example a tablet or capsule containing both a compound of the invention and an analgesic for oral administration, or a twin pack comprising the two active ingredients made up for separate administration.

02 The invention accordingly provides a method of treatment of migraine comprising the administration of an effective amount of a compound of the formula (I) and an analgesic.

03 The invention also provides a compound of formula (I), for use in the treatment of emesis, disorders relating to impaired gastro-intestinal motility and of disorders of the central nervous system.

04 The following examples illustrate the preparation of compounds of the invention and the following descriptions illustrate the preparation of intermediates.

Description 1

5-Dimethylaminosulphonyl-2-fluoro benzoic acid (D1)

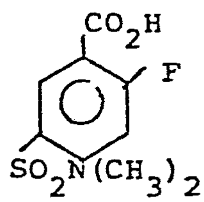

(D1)

Following the procedure outlined in United States Patent 3,843,662, 1972, 2-fluorobenzoic acid (30g) was chlorosulphonated to give 5-chlorosulphonyl-2-fluorobenzoic acid (42.5g, 83%). To the chlorosulphonated product, dissolved in THF (400mls) at 0°, was added aqueous dimethylamine (100mls, ca. 3 molar equivs., 25% w/v solution). The whole was maintained at 0° for a further ½ hour before being poured into water (800mls), acidified, and extracted into methylene chloride (3 x 300mls).

The organic phases were combined, washed with saturated aqueous sodium chloride (100mls), dried ($MgSO_4$), filtered, and concentrated in vacuo. The crude product (42.5g) was triturated with ether to give a white solid (35.4g in two crops) (67%) m.p. 161-2°

n.m.r. (δ, $CDCl_3$) 2.70 (s, 6H, $-N(CH_3)_2$);
7.18-7.31 (m, 1H, H-3);
7.42-7.90 (m, 1H, H-4);
8.20-8.31 (m, 1H, H-6);
10.10 (variable) (br.s., 1H, $-CO_2H$);

Description 2

2-Dimethylamino-5-dimethylaminosulphonylbenzoic acid (D2)

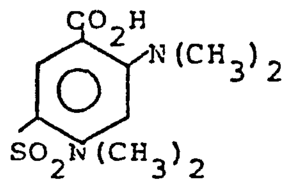

(D2)

5-Dimethylaminosulphonyl-2-fluorobenzoic acid (16g), dissolved in aqueous dimethylamine (100ml, an excess, 25% w/v solution), was heated under reflux for 18 hrs. The solvent was evaporated in vacuo and the residue was dissolved in water (50ml). The pH of the aqueous solution was adjusted to 4-5 and the whole extracted with chloroform (3 x 80ml), dried ($Na_2SO_4$), filtered, and concentrated in vacuo. The solid thus obtained was crystallised from ethyl acetate - petroleum ether (60-80) to give the title compound (11.5g), (62%) m.p. 141-2°.

n.m.r. (δ, $CDCl_3$) 2.77 (s, 6H, $-SO_2N(CH_3)_2$);
2.92 (s, 6H, $-N(CH_3)_2$);
7.7 (d, J = 8Hz, 1H, H-3);
8.0 (dd, J = 2Hz, 1H, H-4);
8.65 (d, J = 2Hz, 1H, H-6):

Description 3

6-Methoxy-isatoic anhydride

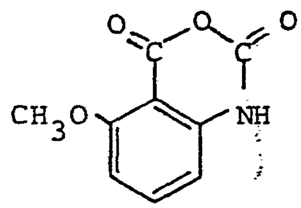

(D3)

6-Methoxy-anthranilic acid (prepared as described by R.N. Warrener et al in Aust. J. Chem. 1980, 33, 2777-9) (3.66g; 0.02 mole) was dissolved in water (25ml) containing sodium carbonate (2.2g). A 12.5% solution of phosgene in toluene (70mls) was added and the mixture was stirred at room temperature overnight. The mixture was filtered to yield the title compound (4.17g) as a pale yellow solid.

mp. 256-8°.

IR ν 3260 $cm^{-1}$ -N-H

Description 4

N-Methyl-6-methoxy isatoic anhydride

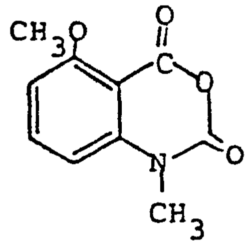

(D4)

6-Methoxy isatoic anhydride (D 3 ) (1.0g; 5.2 mmole) was dissolved in anhydrous dimethylformamide (50ml) under nitrogen. The solution was treated with sodium hydride (140mg; 80% dispersion in oil), left to stir for ½ hour then treated with methyliodide (1ml) (3 x excess) and left overnight at room temperature. The solution was evaporated in vacuo then treated with water to yield a solid, which was filtered and washed with ether (3 x 50ml). Recrystallisation from chloroform-ether gave the title compound (650mg; 61%) as pale yellow crystals.
mp. 218°C.

Following the procedure outlined in description 1, the following benzoic acids were prepared using the appropriate benzoic acids and amines.

2-Bromo-5-dimethylaminosulphonyl benzoic acid (D5)

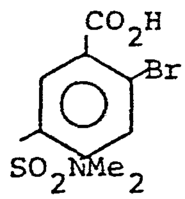

(D5)

Yield = 84% mp 166-9°

n.m.r. [δ, $(CD_3)_2SO$] 2.65 (s, 6H, $-N(CH_3)_2$); 7.50-8.20 (m, 3H, aromatic protons)

m.s. Measured 306.9500 calculated $C_9H_{10}BrNO_4S$ 306.9511

5-Aminosulphonyl-2-bromobenzoic acid (D6)

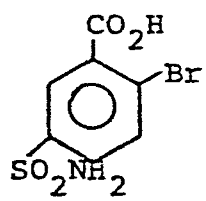

(D6)

Yield = 82%

n.m.r. (δ, $D_6$DMSO), 7.25-8.25 (m, 6H all protons).

m.s. Measured 278.9216 calculated $C_7H_6BrNO_4S$ 278.9198

Example 1

5-Dimethylaminosulphonyl-2-fluoro-N[3'β-(8'-benzyl-8'-azabicyclo{3,2,1}octyl)]benzamide (1)

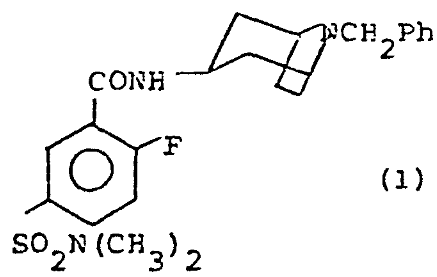

(1)

A solution of 5-dimethylaminosulphonyl-2-fluorobenzoic acid (D1) (7.45g) in thionyl chloride (30 ml), was warmed on a steam bath for 3/4 hour. After cooling to room temperature, the excess thionyl chloride was removed in vacuo and the residue was dissolved in dry toluene (50 ml). The toluene was evaporated in vacuo and the process was repeated. The solid thus obtained was dissolved in dry methylene chloride (100 ml) and cooled to 0°, with stirring. Triethylamine (12 ml), dissolved in dry methylene chloride (30 ml), was added dropwise followed by 3β-8-benzyl-8-azabicyclo{3.2.1}octylamine (6.0g), dissolved in dry methylene chloride (50 ml). Cooling was ceased and the reaction mixture was allowed to warm to room temperature over ca. 2 hr. It was then poured into aqueous potassium carbonate (50 ml) and the organic phase was separated. The aqueous phase was further extracted with methylene chloride (50 ml) and the combined organic phases dried ($K_2CO_3$) and concentrated in vacuo. Trituration of the residue with ether gave a white solid (11.54g) (80%) m.p. 144-50°

n.m.r. (δ, $CDCl_3$) 1.0-2.5 (m, 8H, methylene H's);
2.72 (s, 6H, $-N(CH_3)_2$);
3.2-3.4 (m, 2H, bridgehead H's);
3.56 (s, 2H, $>NC\underline{H}_2Ph$);
4.0-5.0 (m, 1H, 3αH)
6.25-6.75 (m, 1H, $-CON\underline{H}-$);
7.0-7.5 (m, 6H, Ph plus H-3);
7.7-8.0 (m, 1H, H-4);
8.3-8.6 (m, 1H, H-6):

Example 2

2-Dimethylamino-5-dimethylaminosulphonyl-N[3'β-(8'-benzyl-8'-azabicyclo{3.2.1}octyl)]-benzamide (2)

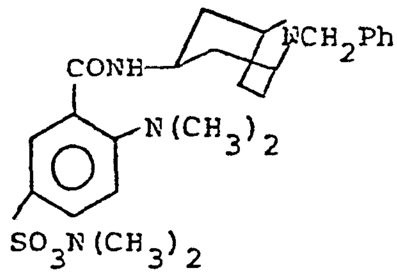

(2)

Following the method outlined in Example 1 2-dimethylamino-5-dimethylaminosulphonylbenzoic acid (D2) (3.8g) was converted to 2-dimethylamino-5-dimethyaminosulphonyl-N-[3'β-(8'-benzyl-8'-azabicyclo{3.2.1}octyl)]-benzamide (2) (4.2g) 64% m.p. 174-6° (ethyl acetate petroleum ether (60-80)).

n.m.r. (δ, $CDCl_3$) 1.5-2.5 (m, 8H, methylene H's);
2.69 (s, 6H, $-SO_2N(CH_3)_2$);
2.84 (s, 6H, $-N(CH_3)_2$);
3.25-3.50 (m, 2H, bridgehead H's);
3.60 (s, 2H, $\equiv NC\underline{H}_2Ph$);
3.8-4.75 (m, 1H, 3αH);
7.1-7.6 (m, 6H, Ph plus H-3);
7.75 (dd, J = 8, 2Hz, 1H, H-4);
7.6-7.9 (m, 1H, -CONH-);
8.14 (d, J = 2Hz, 1H, H-6).

i.r. ($cm^{-1}$, KBr disc) 1 655 (C=O), 3 120-2 640 (br), 3 170 (s), (NH).

m.s. Measured 470.2361 calculated $C_{25}H_{34}N_4O_3S$ 470.2349

Example 3

5-Dimethylaminosulphonyl-2-methylamino-N[3'β(8'-benzyl-8'-azabicyclo{3.2.1}octyl)]benzamide (3)

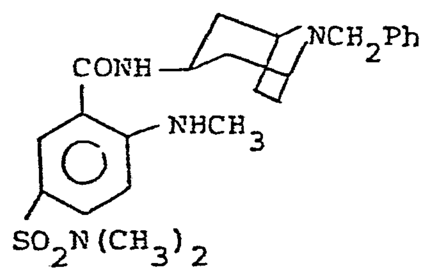

(3)

To 5-dimethylaminosulphonyl-2-fluoro N[3'β(8'benzyl-8'-azabicyclo{3.2.1}octyl)]benzamide (1) (3.5g), dissolved in ethanol (60 ml) was added an ethanolic solution of methylamine (4 molar equiv.), and the whole was heated under reflux for 2½ hr. After cooling to room temperature, the solvent was removed under reduced pressure. The residue was dissolved in chloroform (100ml), washed with brine (50ml), dried ($K_2CO_3$), filtered and concentrated in vacuo. Trituration of the residue with ether gave a white solid which was crystallised from ethyl acetate-petroleum ether (60-80) to give 5-dimethylaminosulphonyl-2-methyl amino-N[3'β-(8'-benzyl-8'azabicyclo{3.2.1}octyl)]benzamide (3) (55%) m.p. 186-8°.

n.m.r. (δ, $CDCl_3$) 1.45-2.40 (m, 8H, methylene H's);
2.65 (s, 6H, $-N(CH_3)_2$);
2.89 (d, J = 5Hz, 3H, $-NHC\underline{H}_3$);
3.15-3.40 (m, 2H, bridgehead H's);
3.56 (s, 2H, $>NC\underline{H}_2Ph$);
4.00-4.70 (m, 1H, 3αH);
5.90-6.25 (m, 1H, -NH-);
6.60-6.75 (m, 1H, H-3);
7.20-7.55 (m, 5H, $>NCH_2\underline{Ph}$);
7.55-7.80 (m, 2H, H-4 + H6);
8.00-8.30 (m, 1H, -NH-):

i.r. ($cm^{-1}$, KBr disc) 1 625 (C=O), 3 120 - 3 525 (br) 3 300 (s) 3 370 (s) ($-N\underline{H}CH_3$).

m.s. Measured 456.2153 calculated $C_{24}H_{32}N_4O_3S$ 456.2191

Example 4

5-Dimethylaminosulphonyl-2-(N-methyl-N-ethoxycarbonyl)amino-N [3'β-(8'-benzyl-8'-azabicyclo{3.2.1}octyl)] benzamide

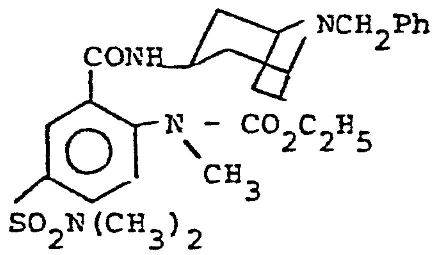

(4)

A solution of 5-dimethylaminosulphonyl-2-methylamino-N[3'β-(8'-benzyl-8'-azabicyclo{3.2.1}octyl)]benzamide (Example 3) (3.1g) and ethyl chloroformate (1ml) in diethyl carbonate (20ml) was heated under reflux for 2hr. On cooling, the reaction mixture was concentrated under reduced pressure and the residue was purified by column chromatography (silica/chloroform), and trituration with ether, to give the 5-dimethylaminosulphonyl-2-(N-methyl-N-ethoxycarbonyl) amino -N[3'β-(8'-benzyl-8'-azabicyclo{3.2.1}octyl)]benzamide (4) (2.9g) (81%) m.p. 148°

n.m.r. (δ, $CDCl_3$) 1.27 (t, J = 7.1Hz, 3H, $-CH_2C\underline{H}_3$);
1.5-2.3 (m, 8H, methylene H's);
2.76 (s, 6H, $-SO_2N(C\underline{H}_3)_2$);
3.0-3.35 (m, 5H, bridgehead H's including 3.24, s, 3H, $>NC\underline{H}_3$)
3.54 (s, 2H, $-NC\underline{H}_2Ph$);
3.9-4.6 (m, 3H. 3α-H including 4.18, q, J = 7.1Hz, 2H, $-CH_2CH_3$);
5.75-6.2 (m, 1H, CON$\underline{H}$);
7.1-7.5 (m, 6H, Ph plus H-3);
7.7-7.95 (m, 2H, H-4 and H-6);

m.s. Measured 528.2387, calculated $C_{27}H_{36}N_4O_5S$ 528.2403

Example 5

5-Dimethylaminosulphonyl-2-ethylamino-N[3'β-(8'-benzyl-8'-azabicyclo{3.2.1}octyl)]-benzamide (5)

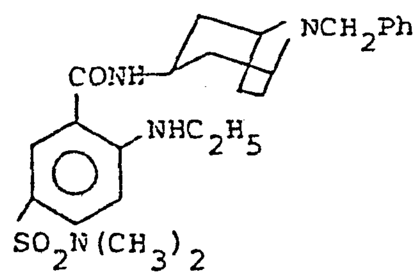

(5)

Following the method outlined in Example 3, 5-dimethylaminosulphonyl-2-fluoro-N[3'β-(8'-benzyl-8'-azabicyclo{3.2.1}octyl)]benzamide (D2) (4g) was converted to 5-dimethylaminosulphonyl-2-ethylamino-N[3'β-(8'benzyl-8'-azabicyclo{3.2.1}octyl)]benzamide (5) (2.23g) (53%) m.p. 197-8°

n.m.r. (δ, $CDCl_3$) 1.30 (t, J = 7Hz, 3H, $-NHCH_2C\underline{H}_3$);
1.40-2.40 (m, 8H, methylene H's);
2.65 (s, 6H, $-SO_2N(CH_3)_2$);
2.80-3.35 (m, 4H, bridgehead H's plus $-NHC\underline{H}_2CH_3$);
3.58 (s, 2H, $>NC\underline{H}_2Ph$);
4.00-4.55 (m, 1H, 3αH);
5.85-6.25 (m, 1H, -NH-);
6.70 (d, J = 6Hz, 1H, H-3);
7.10-7.80 (m, 7H, $>NCH_2P\underline{h}$, plus H-4, H-6);
8.0-8.25 (m, 1H, -NH-):

m.s. Measured 470.2380 calculated $C_{25}H_{34}N_4O_3S$ 470.2349

Example 6

2-Cyclopropylamino-5-dimethylaminosulphonyl-N[-3'β-(8'-benzyl-8'-azabicyclo{3.2.1}octyl)]-benzamide (6)

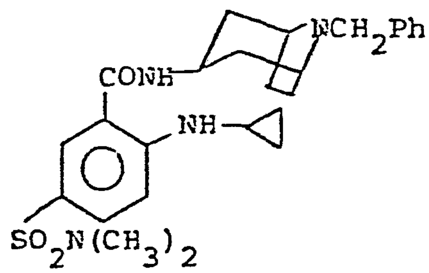

(6)

Following the method outlined in Example 3, 5-dimethylaminosulphonyl-2-fluoro-N[3'β-(8'-benzyl-8'-azabicyclo{3.2.1}octyl)]benzamide (1) (3g) was converted to 2-cyclopropylamino-5-dimethylaminosulphonyl-N[-3'β-(8'-benzyl-8'-azabicyclo{3.2.1}octyl)]benzamide (6) (1.75g) (54%) m.p. 158-60°.

n.m.r. (δ, $CDCl_3$)
0.40-1.00 (m, 4H, cyclopropane methylene H's);
1.40-2.60 (m, 9H, methylene H's plus 1 cyclopropane H);
2.66 (s, 6H, $-SO_2N(CH_3)_2$);
3.20-3.40 (m, 2H, bridgehead H's);
3.58 (s, 2H, $>NC\underline{H}_2Ph$);
3.80-4.55 (m, 1H, 3αH);
5.85-6.25 (m, 1H, -NH-);
7.05-7.50 (m, 6H, $>NCH_2\underline{Ph}$ plus H-3);
7.55-7.80 (m, 2H, H-4, H-6);
8.27 (br s, 1H, -NH-):

Example 7

5-Dimethylaminosulphonyl-2-pyrrolidinyl-N[3'β-(8'-benzyl-8'-azabicyclo{3.2.1}octyl)]-benzamide (7)

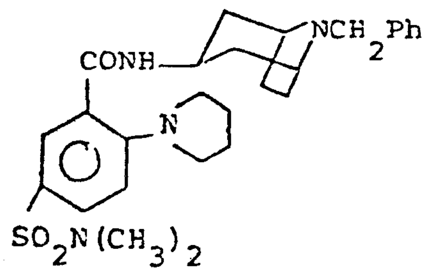

(7)

Following the method outlined in Example 3, 5-dimethylaminosulphonyl-2-fluoro-N-[3'β-(8'-benzyl-8'-azabicyclo{3.2.1}octyl)]benzamide (1). (4g) was converted to 5-dimethylaminosulphonyl-2-pyrrolidinyl-N-(3'β-(8'-benzyl-8'-azabicyclo{3.2.1}octyl)]benzamide (7) (3.3g) (74%) m.p. 212-3°.

n.m.r. (δ, $CDCl_3$) : 1.25-2.25 (m, 12H, methylene H's);
2.63 (s, 6H, $-SO_2N(CH_3)_2$);
3.00-3.75 (m, 6H, bridgehead H's plus 4 pyrrolidine H's);
3.56 (s, 2H, $>NC\underline{H}_2Ph$);
3.95-4.75 (m, 1H, 3αH);
5.60-6.15 (m, 1H, -CONH-);
6.70 (d, J = 9Hz, 1H, H-3);
7.10-7.70 (m, 7H, $>NCH_2\underline{Ph}$ plus H-4, H-6)

m.s. Measured 496.2528 calculated $C_{27}H_{36}N_4O_3S$ 496.2608

Example 8

5-Dimethylaminosulphonyl-2-cyclopentylamino-N-[3β-(8-benzyl-8-azabicyclo {3.2.1} octyl)]benzamide (8)

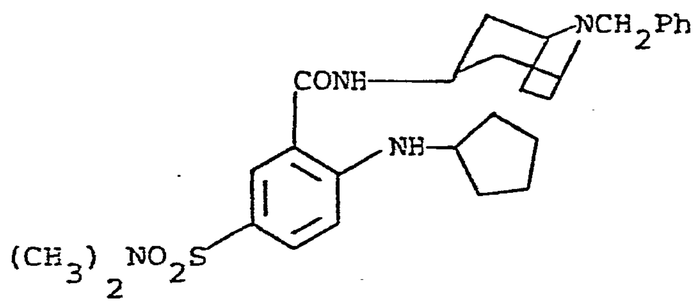

(8)

Following the method outlined in Example 3, 5-dimethylaminosulphonyl-2-fluoro-N-[3β-(8-benzyl-8-azabicyclo {3.2.1} octyl)]benzamide(D2)(4.13g) was converted to the title compound (8) (3g). This was recrystallised from ethyl acetate-petroleum ether (2.6g; 51%) mp 174-6°

| | | |
|---|---|---|
| n.m.r. (δ, $CDCl_3$) | 1.25-2.25 | (m, 16H, methylene H'S) |
| | 2.65 | (s, 6H, $-SO_2N(CH_3)_2$) |
| | 3.25 | (m, 2H, bridgehead H's) |
| | 3.6 | (s, 2H, $>NCH_2Ph$) |
| | 3.8 | (bm, 1H, NH<u>CH</u>) |
| | 4.25 | (bm, 1H, NH<u>CH</u>) |
| | 5.95 | (d, 1H, NH) |
| | 6.7 | (d, 1H, H-3) |
| | 7.2-7.75 | (m, 7H, aromatic protons) |
| | 8.25 | (d, 1H, NH) |

m.s. Measured 510.2663, Calculated for $C_{28}H_{38}N_4O_3S$ 510.2665.

Example 9

5-Dimethylaminosulphonyl-2-cyclobutylamino-N-[3β-(8-benzyl-8-azabicyclo {3.2.1} octyl)]benzamide (9)

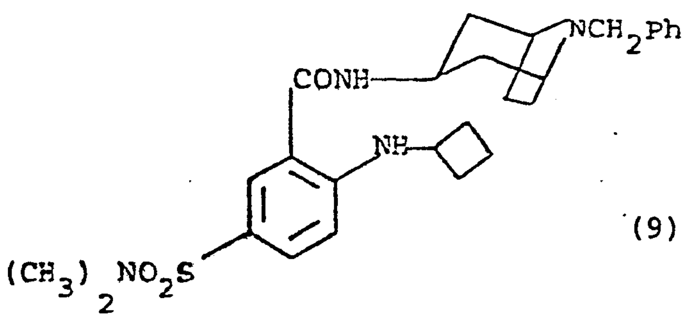

(9)

Following the method outlined in Example 3, but using a longer period at reflux (about 24hrs), 5-dimethylaminosulphonyl-2-fluoro-N-[3β-(8-benzyl-8-azabicyclo {3.2.1} octyl)]benzamide (D2) (2.9g) was converted to the title compound (9) (2.37g). This was recrystallised from ethyl acetate-petroleum ether (1.8g; 52%).

| | | |
|---|---|---|
| n.m.r. (δ, $CDCl_3$) | 1.4-2.6 | (m, 14H, methylene H's) |
| | 2.7 | (s, 6H, $-SO_2N(CH_3)_2$) |
| | 3.3 | (m, 2H, bridgehead H's) |
| | 3.6 | (s, 2H, >$NCH_2Ph$) |
| | 3.95 | (bm, 1H, NHCH) |
| | 4.3 | (bm, 1H, NHCH) |
| | 6.0 | (d, 1H, NH) |
| | 4.6 | (d, H, H-3) |
| | 7.15-7.8 | (m, 7H, aromatic protons) |
| | 8.35 | (d, 1H, NH) |

m.s. Measured 496.2509, Calculated for $C_{27}H_{36}N_4O_3S$ 496.2508.

Example 10

5-Dimethylaminosulphonyl-2-isobutylamino-N-[3β-(8-benzyl-8-azabicyclo {3.2.1} octyl)]benzamide (10)

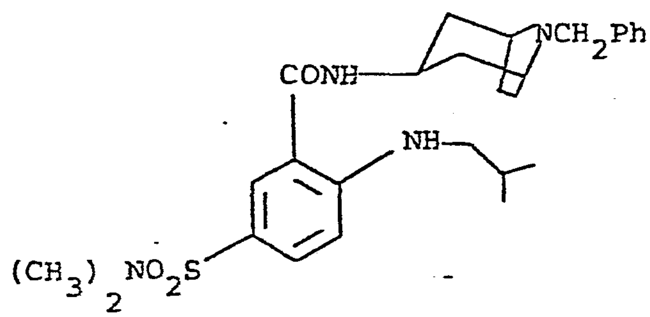

(10)

Following the method outlined in Example 3, but using a longer period at reflux (about 12hr), 5-dimethylaminosulphonyl-2-fluoro-N-[3β-(8-benzyl-8-azabicyclo {3.2.1} octyl)]benzamide (1) (4.13g) was converted to the title compound (10) (3.6g). This was recrystallised from ethyl acetate-petroleum ether (2.4g; 48%)

| | | |
|---|---|---|
| n.m.r. (δ, $CDCl_3$) | 1.0 | (d, 6H 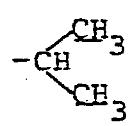 ) |
| | 1.4-2.35 | (m, 9H, methylene H's) |
| | 2.68 | (s, 6H, $-SO_2N(CH_3)_2$) |
| | 3.0 | (t, 2H, NH$\underline{CH_2}$) |
| | 3.25 | (m, 2H, bridgehead H's) |
| | 3.59 | (s, 2H, $>NCH_2Ph$) |
| | 4.25 | (bm, 1H, NHCH) |
| | 5.95 | (d, 1H, NH) |
| | 6.67 | (d, 1H, H-3) |
| | 7.15-7.75 | (m, 7H, aromatic protons) |
| | 8.3 | (m, 1H, NH) |

m.s. Measured 498.2657, Calculated for $C_{27}H_{38}N_4O_3S$ 498.2665.

Example 11

5-Dimethylaminosulphonyl-2-isopropylamino-N-[3β-(8-benzyl-8-azabicyclo {3.2.1} octyl)]benzamide (11)

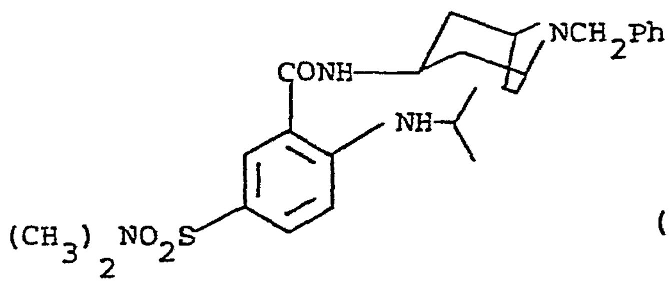

(11)

Following the method outlined in Example 3, but using a longer period at reflux (about 24hrs), 5-dimethylaminosulphonyl-2-fluoro-N-[3β-(8-benzyl-8-azabicyclo {3.2.1} octyl)]benzamide (1) (4.13g) was converted to the title compound (11) (4.1g). This was recrystallised from ethyl acetate-petroleum ether (2.7g; 56%).

| | | |
|---|---|---|
| n.m.r. (δ, $CDCl_3$) | 1.26 | (d, 6H, $CH(\underline{CH_3})_2$ |
| | 1.45-2.25 | (m, 8H, methylene H's) |
| | 2.68 | (s, 6H, $-SO_2N(CH_3)_2$) |
| | 3.25 | (m, 2H, bridgehead H's) |
| | 3.6 | (s, 2H, >$NCH_2Ph$) |
| | 3.7 | (bm, 1H, NH$\underline{CH}$) |
| | 4.3 | (bm, 1H, NH$\underline{CH}$) |
| | 6.0 | (d, 1H, NH) |
| | 6.7 | (d, 1H, H-3) |
| | 7.1-7.8 | (m, 7H, aromatic H's) |
| | 8.18 | (d, 1H, NH) |

m.s. Measured 484.2509, Calculated for $C_{26}H_{36}N_4O_3S$ 484.2508.

Example 12

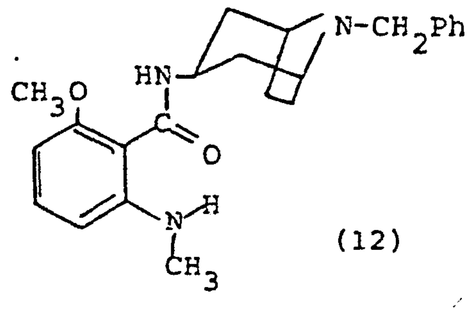

(12)

2-Methylamino-6-methoxy-N-[3β-(8-benzyl-8-azabicyclo (3,2,1)octyl)]benzamide (12)

N-Methyl-6-methoxyisatoic anhydride (D 4 ) (3.33g, 0.016 mole); anhydrous dimethylformamide (50ml) and N-benzyl-nortropane-3-β-amine (3.5g) were heated to 50° for 1 hour with stirring then left overnight at room temperature. The mixture was evaporated in vacuo, the residue was taken up in chloroform and chromatographed on Kieselgel 7734 to give the title compound (3.21g; 53%) as colourless microcrystals mp 162-163° (ex ethyl acetate-ether).

$C_{23}H_{29}N_3O_2$

Requires % C = 72.82, H = 7.65, N = 11.08

Found % C = 72.63, H = 7.46, N = 11.18

Calculated $M^+$ = 379.2260, observed $M^+$ = 379.2263

Example 13

2-Bromo-5-dimethylaminosulphonyl-N[3β-(8-benzyl-8-azabicyclo{3.2.1}octyl)]benzamide (13)

Following the procedure outlined in description 2 2-bromo-5-dimethylaminosulphonyl benzoic acid ( D5 ) (5g) was converted to 2-bromo-5-dimethylaminosulphonyl-N[3-(8-benzyl-8-azabicyclo{3.2.1}octyl)]benzamide (Compound 13 ) (4.53g) (79%) m.p. 167-8°.

n.m.r. (δ, $CDCl_3$) 1.30-2.50 (m, 8H, methylene protons);
2.70 (s, 6H, $-N(CH_3)_2$);
3.00-3.40 (m, 2H, methyne protons);
3.55 (s, 2H, $>NC\underline{H}_2Ph$);
4.00-4.65 (m, 1H, 3αH);
5.75-6.05 (m, 1H, -CON$\underline{H}$-);
7.1 -7.9 (m, 8H, aromatic protons)

i.r. (νmax, KBr) 1685 (C=O) 3260 (NH) $cm^{-1}$
m.s. Measured 505.0996 calculated $C_{23}H_{28}BrN_3O_3S$ 505.1033

| | Theoretical | Found | |
|---|---|---|---|
| C | 54.54 | 54.75 | 54.73 |
| H | 5.57 | 5.65 | 5.64 |
| N | 8.30 | 8.28 | 8.32 |
| S | 6.33 | 6.49 | 6.40 |
| Br | 15.78 | 15.66 | |

Example 14

5-Aminosulphonyl-2-methyl-N[3β(8-benzyl-8-azabicyclo{3.2.1} octyl)]benzamide (14)

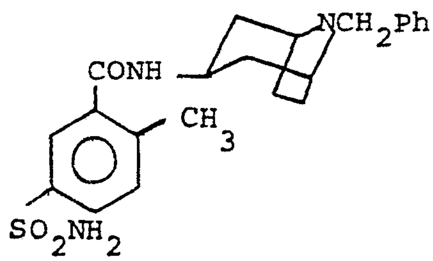

(14)

5-Aminosulphonyl-2-methylbenzoic acid (2.0g) was dissolved in dry dimethylformamide (30mls) and triethylamine (1.25mls) was added before cooling to 0°C. Ethylchloroformate (0.86ml) was added dropwise to the reaction mixture whilst keeping the temperature at 0°C, and the mixture was stirred at this temperature for 15 minutes.

3β-Amino-8-benzyl-8-azabicyclo{3.2.1}octane (1.94g) in dry dimethylformamide (5mls) was added in one portion to the reaction mixture at 0°C before stirring at room temperature for 15 hours.

The solvent was removed in vacuo to leave an oil which, on addition of ammonia solution, gave a white solid (1.64g). Treatment of this solid with ether gave the desired product ( 14 ) (1.4g, 38%)

m.p. 185-7°C

n.m.r. (δ, $(CD_3)_2SO$) 2.45 (3H, s, $ArCH_3$)
4.05-4.65 (3H, m + s, 3αH, $NCH_2Ph$)
6.9 -7.95 (8H, m, aromatic H's)
8.45 (1H, d, CONH)

m.s. measured 413.1768; Calculated for $C_{22}H_{27}N_3SO_3$ 413.1770

The following compounds were prepared analogously. 5-Dimethylaminosulphonyl-2-methyl-N-[3β-(8-benzyl-8-azabicyclo 3.2.1 octyl)]benzamide (76% yield; m.p. 184-5)

(Compound 15)

n.m.r. (δ, $(CD_3)_2SO$) 1.4-2.15 (8H, m, methylene H's)
2.4 (3H, s, $ArCH_3$)
2.6 (6H, s, $N(CH_3)_2$)
3.15 (2H, m, bridgehead H's)
3.3 (2H, s, $NCH_2Ph$)
4.15 (1H, m, 3αH)
7.1-7.8 (8H, m, aromatic H's)
8.3 (1H, d, -CONH)

5-Aminosulphonyl-2-bromo-N[3β-(8-benzyl-8-azabicyclo {3.2.1}octyl)]benzamide which was converted to its hydrochloride salt via standard methods. (63% yield; m.p. 190-5)

(Compound 16)

n.m.r. (δ, $(CD_3)_2SO$) 1.6-2.2 (8H, m, methylene H's)
3.0-3.5 (2H, m, bridgehead H's)
3.5-3.9 (2H, m, $SO_2NH_2$)
3.9-4.6 (2H, m, $NCH_2Ph$)
7.25-8.0 (8H, m, aromatic H's)
8.6-9.0 (1H, m, CONH)

m.s. Measured 477.0733, calculated for $C_{21}H_{24}BrN_3O_3S$ 477.0719

Pharmacological Data

Gastric Motility Enhancing Activity

i) Increase in intragastric pressure

Intragastric pressure changes were recorded from previously starved conscious but restrained rats using a saline filled catheter inserted into the lumen of the stomach via a permanent gastric fistula. The catheter was connected to a physiological pressure transducer and pressure changes recorded on a hot wire pen recorder. In each animal a pre-dose period of 40 minutes was allowed to obtain a measure of spontaneous activity. An index of activity was obtained by measuring the average height of pressure waves during 10 minute periods. Values for 4 such periods were obtained during assessment of spontaneous activity and for 40 minute period after administration of compound. Student's ''t'' test was applied to the difference in average values obtained for spontaneous and post compound activity.

The compounds of Examples 3, 5 and 6 significantly increased index of activity post administration at a dose of 0.5 mg/kg s.c.

ii) Heidenhain Pouch Test

Male Beagle dogs (12-15 kg) with a Heidenhain pouch were placed in slings in Pavlov stands. Experiments wre conducted at least 18 hours after the animals had last been fed. The motility of the pouch was monitored via an open-tipped catheter inserted via the fistula and connected to a Bell and Howell (type 4-4 22) pressure transducer. After suitable amplification

the motility of the pouch was displayed on a hot wire pen recorder (Ormed MX216). Drugs were administered intravenously via a suitable peripheral vein.

The compounds of Examples 2 and 6 were active at a dose of 0.5 mg/kg s.c. The compound of Example 3 was active at a dose of 0.1 mg/kg s.c. The compounds of Examples 3 and 6 were active for a period of greater than 1 hour.

Anti-emetic activity in the dog

Compounds were administered subcutaneously 30 minutes prior to administration of a standard dose of apomorphine HCl (0.1 mg/kg subcutaneously) and the vomiting response compared to that obtained when the same animals were dosed with apomorphine HCl and vehicle only. The compound of Example 2 had an $ED_{50}$ value of 0.1 mg/kg s.c. and the compound of Example 6 had an $ED_{50}$ value of 0.5 mg/kg s.c. The compound of Example 12 was active at a dose of 0.01 mg/kg s.c.

Dopamine receptor blocking activity in the central nervous system

Compounds were tested for inhibition of apomorphine-induced climbing in the mouse. The test is based on that described by Protais, P., Constantin, J. and Schwartz J.C. (1976), Psychopharmacology, 50, 1.6.

Apomorphine 1 mg/kg s.c. induces mice to climb the wall of a wire cage (inverted food hopper - 11 x 7.5 x 18 cm high). Mice acclimatised in their home cages in groups of 5 are placed under the hoppers immediately after the injection of apomorphine 1 mg/kg s.c. At 10.20 and 30 minutes after injection climbing behaviour is scored. The mice are observed for 30 seconds and scored according to the position they spend the

majority of time in, score 0 - four paws on floor of cage; score 1 - fore paws only on walls; score 2 - all paws on wall of cage. The scores at all 3 times and for each mouse are summed and mice drug treated orally compared to mice receiving apomorphine only. A saline only treated group is also included and any score 55% of maximum taken into account.

The compounds of Examples 2, 4, 5 and 6 were inactive at a dose of 10 mg/kg s.c. The compound of Example 3 was inactive at a dose of 25 mg/kg s.c.

Toxicity

No toxic effects were observed in the above tests.

## Claims

1. A compound of formula (I), or a pharmaceutically acceptable salt and/or N-oxide and/or a solvate thereof:

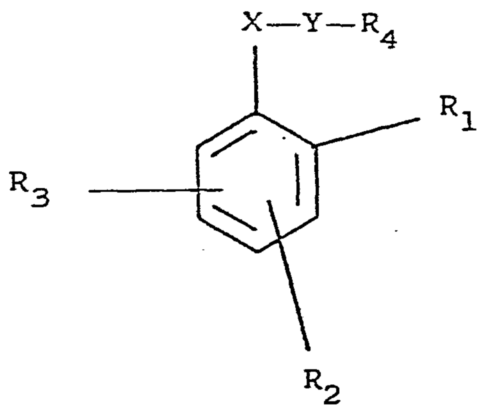

(I)

wherein:

one of X and Y is CO and the other is NH;

$R_4$ is a group:

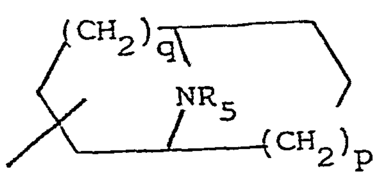

(II)

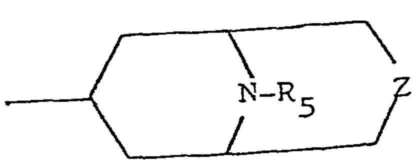

(III)

or

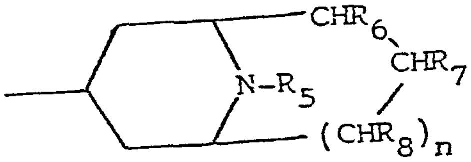

(IV)

wherein:

p and q each independently are 0 to 2;
Z is O or S;
n is 0 or 1;
one of $R_6$ and $R_7$ when n=0 is $C_{1-4}$ alkoxy, $C_{1-4}$ alkoxycarbonyl, hydroxy or $C_{1-4}$ alkyl optionally substituted by hydroxy, $C_{1-4}$ alkoxy or $C_{1-4}$ acyloxy. and the other is hydrogen or $C_{1-4}$ alkyl or one of $R_6$, $R_7$ and $R_8$ when n=1 is $C_{1-4}$ alkyl and the other two are the same or different and are hydrogen or $C_{1-4}$ alkyl;

$R_5$ is $C_{1-7}$ alkyl, - $(CH_2)_sR_{10}$,s being 0 to 2 and $R_{10}$ being $C_{3-8}$ cycloalkyl, $-(CH_2)_tR_{11}$,t being 1 or 2 and $R_{11}$ being thienyl or phenyl optionally substituted by one or two substituents selected from $C_{1-4}$ alkoxy, trifluoromethyl, halogen, nitro, carboxy, esterified carboxy and $C_{1-4}$ alkyl optionally substituted by hydroxy, $C_{1-4}$ alkoxy, carboxy, esterified carboxy or in vivo hydrolysable acyloxy;

$R_1$ is carboxylic $C_{1-7}$ acyl,carboxylic $C_{1-7}$ acylamino optionally N-substituted by $C_{1-6}$ alkyl, carboxylic $C_{1-7}$ acyloxyamino, $C_{1-6}$ alkylsulphonyl, $C_{1-6}$ alkylsulphinyl, halogen, $C_{1-6}$ alkyl, $C_{3-6}$ cycloalkyl or amino optionally substituted by one or two groups selected from $C_{1-6}$ alkyl, $C_{3-8}$ cycloalkyl, $C_{3-8}$ cycloalkyl $C_{1-4}$ alkyl, phenyl or phenyl $C_{1-4}$ alkyl groups any of which phenyl moieties may be substituted by one or more halogen, trifluoromethyl, $C_{1-6}$ alkoxy or nitro groups, or amino-disubstituted by $C_{4-5}$ polymethylene; and

$R_2$ and $R_3$ are the same or different and are hydrogen, halogen, trifluoromethyl, $C_{1-6}$ alkoxy, $C_{1-6}$ alkylthio, carboxylic $C_{1-7}$ acyl, carboxylic $C_{1-7}$ acylamino, $C_{1-6}$ alkylsulphonyl, $C_{1-6}$ alkylsulphinyl,

nitro or $NR_{12}R_{13}$, $NR_{12}R_{13}CO$, $NR_{12}$ $R_{13}SO_2$, or $C_{1-6}$ alkyl-$SO_2NR_{14}$, $NR_{12}R_{13}SO_2NR_{14}$ wherein $R_{12}$ and $R_{13}$ are the same or different and are hydrogen, $C_{1-6}$ alkyl, $C_{3-8}$ cycloalkyl, phenyl or phenyl $C_{1-4}$ alkyl groups any of which phenyl moieties may be substituted by one or more halogen, trifluoromethyl, $C_{1-6}$ alkoxy or nitro groups, or $R_{12}$ and $R_{13}$ together form $C_{4-5}$ polymethylene, and $R_{14}$ is hydrogen or $C_{1-6}$ alkyl.

2. A compound according to claim 1 of formula (VI):

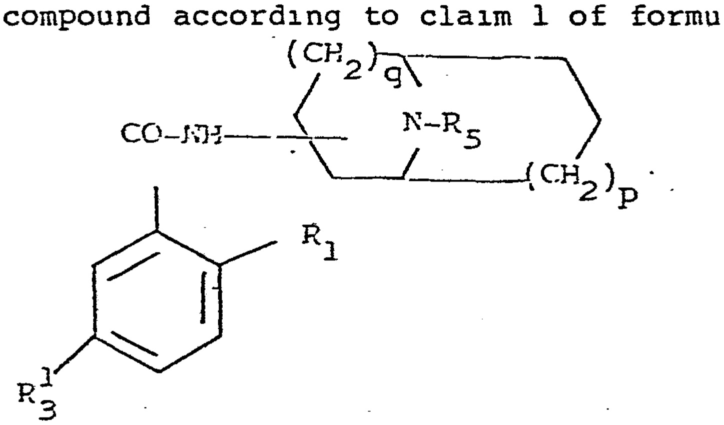

(VI)

wherein $R_3^1$ is aminosulphonyl optionally substituted as difined for $R_3$ in claim 1 and the remaining variables are as defined in claim 1.

3. A compound according to claim 2 of formula (VIII):

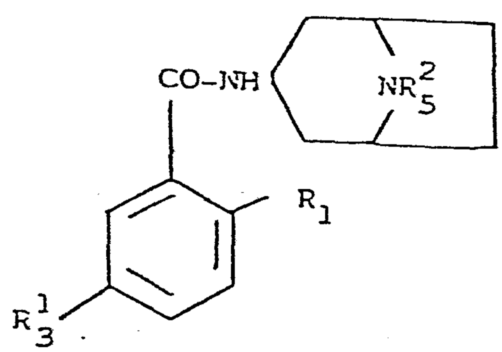

(VIII)

wherein:

$R_2^5$ is thienylmethyl or $-(CH_2)_tR_{11}$, t being 1 or 2, and $R_{11}$ being phenyl optionally subsituted by one or two substituents selected from $C_{1-4}$ alkoxy, trifluoromethyl, halogen, carboxy, esterified carboxy, and $C_{1-4}$ alkyl optionally substituted by hydroxy, $C_{1-4}$ alkoxy, carboxy, esterified carboxy or in vivo hydrolysable acyloxy, and the remaining variables are as defined in claims 1 and 2.

4. A compound according to claim 1 of formula (XIII):

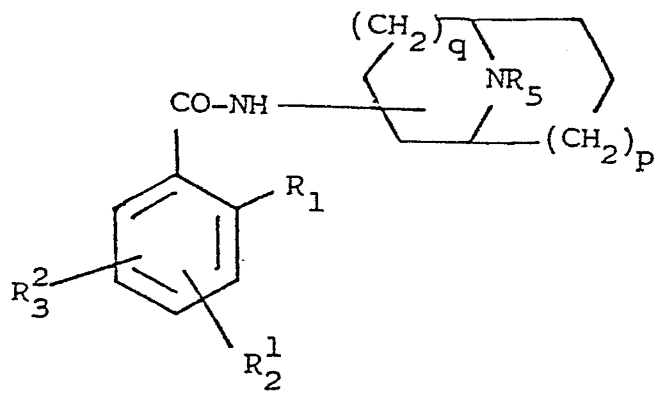

(XIII)

wherein $R_1^2$ and $R_2^3$ are the same or different and are hydrogen, halogen, hydroxy, $C_{1-6}$ alkoxy, $C_{1-6}$ alkylthio, trifluoromethyl, amino or $C_{1-7}$ acylamino; and the remaining variables are as defined in claim 1.

5. A compound according to claim 4 of formula (XV)

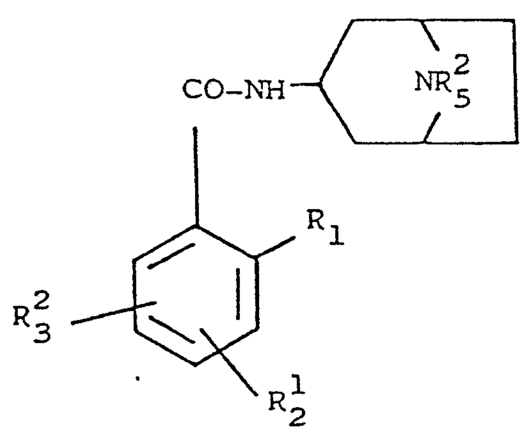

(XV)

wherein $R_1$ is a defined in claim 1;
$R_2{}^1$ and $R_3{}^2$ are as defined in claim 4; and
$R_5{}^2$ is as defined in claim 3.

6. A compound according to any one of claims 1 to 5 wherein $R_1$ is mono- or di-substituted amino as defined in claim 1.

7. A compound according to any one of claims 1 to 6 wherein $R_5$ or $R_5{}^2$ is benzyl.

8. 5-Dimethylaminosulphonyl-2-fluoro-N[3'β-(8'-benzyl -8'-azabicyclo[3,2,1]octyl)]benzamide,

2-Dimethylamino-5-dimethylaminosulphonyl-N[3'β-(8' benzyl-8'-azabicyclo[3.2.1.]octyl)]-benzamide,

5-Dimethylaminosulphonyl-2-methylamino-N[3'β(8'-benzyl-8'-azabicyclo[3.2.1]octyl)]benzamide,

5-Dimethylaminosulphonyl-2-(N-methyl-N-ethoxycarbonyl)amino-N[3'β-(8'-benzyl-8'-azabicyclo [3.2.1]octyl)]benzamide,

5-Dimethylaminosulphonyl-2-ethylamino-N[3'β-(8'-benzyl-8'azabicyclo[3.2.1]octyl)]benzamide,

2-Cyclopropylamino-5-dimethylaminosulphonyl-N [-3'β-(8'-benzyl-8'-azabicyclo[3.2.1]octyl)] benzamide,

5-Dimethylaminosulphonyl-2-pyrrolidinyl-N[3'β-(8'-benzyl-8'-azabicyclo[3.2.1]octyl)]benzamide,

5-Dimethylaminosulphonyl-2-cyclopentylamino-N-[3β-(8-benzyl-8-azabicyclo[3.2.1]octyl)]benzamide,

5-Dimethylaminosulphonyl-2-cyclobutylamino-N-[3β-(8-benzyl-8-azabicyclo[3.2.1]octyl)]benzamide,

5-Dimethylaminosulphonyl-2-isobutylamino-N-[3β-(8-benzyl-8-azabicyclo[3.2.1]octyl)]benzamide,

5-Dimethylaminosulphonyl-2-isopropylamino-N-[3β-(8-benzyl-8-azabicyclo[3.2.1]octyl)]benzamide,

6-Methoxy-2-methylamino-N-[3β-(8-benzyl-8-azabicyclo[3.2.1]octyl)]benzamide

or

2-Methylamino-6-methoxy-N-[3β-(8-benzyl-8-azabicyclo[3.2.1]octyl)]benzamide

9. A process for the preparation of a compound according to any one of claims 1 to 8 which process comprises reacting a compound of formula (XIX):

with a compound of formula (XX):

$$L-R_4 \qquad (XX)$$

wherein:

One of J and L is COQ, where Q is a leaving group, and the other is $-NH_2$; and the remaining variable groups are as defined in claim 1, with the proviso that when J is $-NH_2$, $R_1$, $R_2$ or $R_3$ is other than amino, and thereafter optionally converting $R_1$, $R_2$ or $R_3$ to another $R_1$, $R_2$ or $R_3$ respectively; as necessary converting $R_5$ to other $R_5$; and optionally forming a pharmaceutically acceptable salt of the resultant compound of the formula (I).

10. A pharmaceutical composition comprising a compound according to any one of claims 1 to 8 and a pharmaceutically acceptable carrier.

11. A compound according to any of claims 1 to 8 for use in treating disorders relating to impaired gastro-intestinal motility and/or emesis in mammals.